# EUROPEAN PATENT SPECIFICATION

(11) **EP 1 984 520 B1**
(45) Date of publication and mention of the grant of the patent: **11.12.2013**
(21) Application number: 07704973.2
(22) Date of filing: 23.01.2007
(51) Int. Cl.: C12Q 1/68

(54) **HIGH THROUGHPUT TESTING FOR PRESENCE OF MICROORGANISMS IN A BIOLOGICAL SAMPLE**
TESTEN MIT HOHEM DURCHSATZ AUF DAS VORHANDENSEIN VON MIKROORGANISMEN IN EINER BIOLOGISCHEN PROBE
RECHERCHE A HAUT DEBIT DE MICRO-ORGANISMES DANS UN ECHANTILLON BIOLOGIQUE

(30) Priority: 23.01.2006 GB 0601302
(43) Date of publication of application: 29.10.2008
(73) Proprietor: Rochester Investment Partners, London WC1N 3XX (GB)
(72) Inventor: SEMIKHODSKII, Andrei, Wimbledon SW19 5HP (GB); GREEN, Simon, Wimbledon SW19 5HP (GB)
(74) Representative: Hopley, Joanne Selina
(86) International application number: PCT/GB2007/000195
(87) International publication number: WO 2007/083147

(56) References cited:
- EP-A- 1 473 370
- WO-A-93/24659
- WO-A-94/03634
- DE-A1- 19 945 916
- JP-A- 2002 281 980
- US-A- 5 654 418
- US-A1- 2002 187 470
- BECKER ET AL.: "Real-time PCR for detection of Trypanosoma brucei in human blood samples" DIAGNOSTIC MICROBIOLOGY AND INFECTIOUS DISEASE, vol. 50, 2004, pages 193-199, XP002444026
- NDUNGURU ET AL.: "Application of FTA technology for sampling, recovery and molecular characterization of viral pathogens and virus-derived trangenes from plants tissues" VIROLOGY JOURNAL, vol. 2, 2005, pages 1-12, XP002444027
- DORE MARIA P ET AL: "Helicobacter infection in patients with HCV-related chronic hepatitis, cirrhosis, and hepatocellular carcinoma" DIGESTIVE DISEASES AND SCIENCES, vol. 47, no. 7, July 2002 (2002-07), pages 1638-1643, XP002445132 ISSN: 0163-2116
- KWON ET AL: "Rapid detection of probiotic Lactobacillus species by multipex PCR using species-specific primers based on the region extending from 16S rRNA through 23S rRNA" FEMS MICROBIOLOGY LETTERS, vol. 239, 2004, pages 267-275, XP002445133
- YOSHIDA TAKASHI ET AL: "Rapid detection of Mycoplasma genitalium, Mycoplasma hominis, Ureaplasma parvum, and Ureaplasma urealyticum organisms in genitourinary samples by PCR-microtiter plate hybridization assay." JOURNAL OF CLINICAL MICROBIOLOGY MAY 2003, vol. 41, no. 5, May 2003 (2003-05), pages 1850-1855, XP002444074 ISSN: 0095-1137

## Description

### FIELD

The invention relates to high throughput multiplex testing for microorganisms that may be present in a biological sample using nucleic acid based enzymatic techniques. More specifically, the invention is directed towards identification of pathogenic microorganisms.

### BACKGROUND

Since the identification in the nineteenth century of microorganisms as one of the major sources of morbidity and mortality, efforts have continued to monitor and control the spread of infectious disease. The earliest efforts were made by pioneers in the young science of epidemiology such as Dr John Snow, who famously removed the handle of London's Broad Street Pump after he identified it as the source of the City-wide cholera outbreak. In the twentieth century, the advent of antibiotics, mass vaccination and antiviral treatments has offered an unprecedented level of control over the spread of such diseases, in the developed world at least. Nevertheless, infectious disease still remains one of the main causes of death worldwide, with an unrelenting succession of 'new' microbial killers seemingly emerging every year. MRSA, SARS, avian flu, HIV and malaria represent but a few of the many infectious pathogens causing alarm and concern around the world.

International health organisations such as the UN and the WHO consistently express concern over the unrestrained use of antibiotic compounds, leading to increasing levels of antibiotic resistance amongst many microbial species. In addition sexually transmitted infections (STIs) represent one of the greatest infectious disease problems in the world today and in some regions, particularly Africa and the former Soviet Union, are at epidemic level. According to one study (Adler, M., 2005, Why sexually transmitted infections are important. In: ABC of Sexually Transmitted Infections. 5th ed. BMJ Books) the number of reported infected people in Western Europe was 17 million, in the USA it was 15 million, in Africa 70 million, and globally around 400 million.

Combating sexually transmitted infections and HIV/AIDS remains uppermost on the world's governmental health agendas and the need for improvement in access to health services plus the creation and provision of diagnostic services available for all who need them are clear. Many STIs are asymptomatic and can only be diagnosed through testing, however routine screening programmes are extremely rare, social stigma high, funding inadequate and public awareness limited.

The effects of infectious diseases are not limited to the human population, severe economic damage is caused by outbreaks of diseases in livestock and plant crops. Outbreaks of swine fever in pigs, foot and mouth disease in cattle and avian flu can rapidly spread and decimate the agricultural output and the economy of a country. In the UK in 2001, an outbreak of foot and mouth disease lead to the culling of over seven million cattle and sheep as well as the effective 'closure' of rural areas.

Currently available systems for screening for the presence of microorganisms in a sample taken from a host (such as a patient, animal or plant source) are low throughput. In the clinical environment, usually only one microorganism is tested for per sample unless there are medical indications that the host may suffer from several microbial diseases. This means that it is usually only possible to detect a single infection per test, which has a significant impact on the price and the testing time. In addition, in an asymptomatic host it is often difficult to decide which microorganism the patient should be tested for. Negative results for two or three infectious organisms can provide a false sense of security.

Modern detection systems used in clinical practice employ DNA based assays for detection of microorganism infection. The most common methodologies are based on Polymerase Chain Reaction (PCR), Ligase Chain Reaction, Strand Displacement Amplification, Transcription Mediated Amplification, Sequence Based Amplification Assay and several others. These approaches are also low throughput, time consuming, require a large amount of hands-on time and are difficult to automate. In addition to this, there remains no reliable approach which could be used to detect multiple pathogens in a single test, using a single sample taken from the host organism.

Hence, it would be desirable to provide a means for reliably and cheaply testing tor a plurality of microorganisms that may be present in a biological sample. In particular it would be desirable to provide means for effecting testing that can allow for reliable biological sample collection in home or otherwise in the field.

### SUMMARY

In broad terms, the present invention overcomes the aforementioned problems in the prior art by providing methods for high throughput testing of biological samples that may or may not comprise microorganisms. The methods include the use of a diagnostic multiplexing panel (DMP) specifically designed for the simultaneous identification of a plurality of potential microorganism species that may or may not be present in the biological sample.

Accordingly, the invention provides a method for determining whether two or more species of specified microorganisms are present within a biological sample that potentially comprises the microorganisms comprising:
(a) immobilizing the biological sample on and/or within a solid substrate at a first location;
(b) transferring the immobilized biological sample to at least a second location and performing a extraction step on the solid substrate so as to extract any microorganism DNA immobilized on and/or within the solid substrate;
(c) performing a nucleic acid amplification step on microorganism DNA extracted in step (b), wherein the amplification step comprises a plurality of amplification primers that are directed towards amplification of a plurality of highly conserved sequences, from the two or more specific microorganisms, and wherein amplified sequences are designated as target sequences;
(d) combining the target sequences with a plurality of primer sequences comprised within a diagnostic multiplexing panel (DMP), wherein each primer sequence facilitates genotyping of the target sequence;
(e) performing a primer extension reaction on the combination of target sequences and the DMP present in (d), thereby producing a DMP reaction product; and
(f) analysing the reaction product so as to determine genotype of any target sequences that are present and correlating the genotype of the target sequences in the reaction product with the identification of specified microorganisms present in the biological sample;
wherein the specified microorganisms are selected from one or more of the group consisting of: Mycoplasma spp., Chlamydia spp., Ureaplasma spp., Neisseria spp., Gardnerella spp., Trichomonas spp., and Treponema spp., and Candida albicans;
and wherein the DMP comprises one or more primer sequences selected from SEQ ID NOs: 31-45, and/or one or more primer sequences selected from SEQ ID NOs: 76-90.

Also described is a DMP, suitable for use in genotyping pathogenic microorganisms known to cause at least one infectious disease that may be present within a biological sample, the DMP comprising a plurality of primer sequences directed at identification of at least two or more SNPs present in a highly conserved allele of at least one microorganism known to cause an infectious disease when used in a primer extension reaction.

Also described herein is a microorganism testing kit suitable for personal use by a user located in a first location, the kit comprising a testing surface located within a sealable chamber, the testing surface further comprising a solid substrate that is capable of immobilizing a biological sample either within it or upon its surface, and wherein once a biological sample is deposited upon the testing surface, the chamber is sealed around the testing surface such that the testing kit can be despatched to a second location for analysis to determine whether one or more microorganisms are present in the biological sample.

The diagnostic method of the invention may be useful in the treatment of an animal, including a human, that is suspected of carrying one or more infectious microorganisms, by obtaining a biological sample from the animal, testing the biological sample according to the methods described herein, thereby diagnosing whether the animal is infected with one or more infectious microorganisms, and administering treatment to the animal, which treatment is configured appropriately in light of the information regarding the type(s) of infectious microorganisms found to be present in the biological sample.

These and other uses, features and advantages of the invention should be apparent to those skilled in the art from the teachings provided herein.

### DRAWINGS

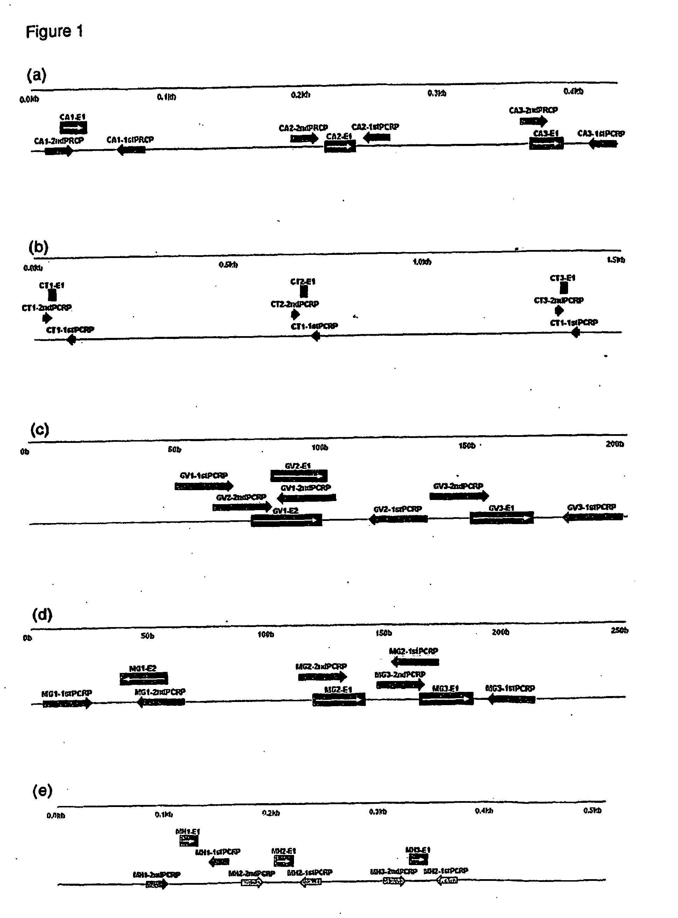

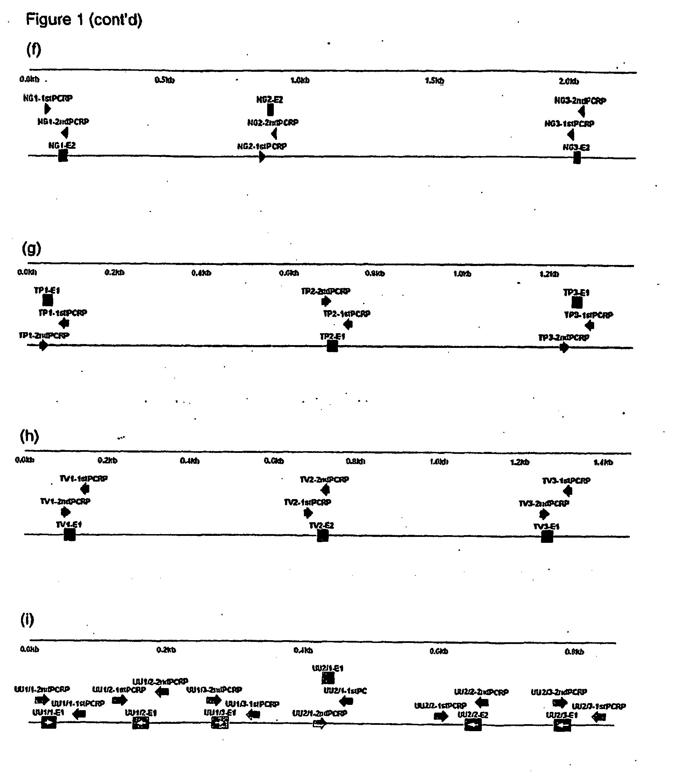

Figure 1 shows a schematic representation of highly conserved DNA consensus sequences - the consensus sequences were generated by comparison of several strains of the same species - the locations at which primers for the amplification or primer extension reactions are selected for use in a DMP of the invention are shown. For each primer, the first two letters identify the organism, the letter after identifies the target sequence for the species (1-3); 1stPCRP denotes the first PCR primer; 2ndPCRP denotes the second PCR primer; E1 or E2 denotes an extension primer; for Ureaplasma there are two sites for which the primers were designed (UU1 and UU2). The identity of the primers is set out in Table 1. The organisms are (a) Candida albicans; (b) Chlamydia trachomatis; (c) Gardnerella vaginalis; (d) Mycoplasma genitalium; (e) Mycoplasma hominis; (f) Neisseria gonorrhoea; (g) Treponema pallidum; (h) Trichomonas vaginalis; (i) Ureaplasma urealyticum.

### DETAILED DESCRIPTION

In setting forth the invention, a number of definitions are provided that will assist in the understanding of the invention. Unless otherwise defined, all technical and scientific terms used herein have the same meaning as commonly understood by one of ordinary skill in the art to which this invention belongs. Where common molecular biology techniques are described it is expected that a person of skill in the art would have knowledge of such techniques, for example from standard texts such as Sambrook J. et al, (2001) Molecular Cloning: a Laboratory Manual, Cold Spring Harbor Press, Cold Spring Harbor, NY.

The term "specified microorganisms" as used herein is intended to denote one or more specified species of microorganism that may or may not be present in a biological sample. The specified microorganisms are suitably of viral, bacterial, fungal (including unicellular yeast) and/or protozoan (including plasmodium) origin. Typically, the specified microorganisms will be pathogenic to an animal host at some point in their life cycle. However, the present invention is adequate for testing for species of microorganisms that exhibit dormancy, commensal infection or sub-clinical infection.

The term "biological sample" as used herein is intended to encompass samples that may contain one or more of specified microorganisms that are tested for according to the present invention. Depending upon the intention of the DMP, the biological sample may be obtained from a human patient, a non-human animal, from a plant or from a foodstuff. In the latter case the DMP will be intended for the purpose of determining contamination of the foodstuff by food-borne pathogens. The samples can suitably include, for example, urine; faeces; vaginal, nasal, rectal or oral swabs; blood, saliva; and/or sputum; semen; vaginal or urethral discharges and swabs thereof; tears (i.e. lacrimal secretions); biopsy tissue samples; and swabs of surfaces upon which any of the aforementioned secretions and substances may have been deposited. Whilst the biological sample of the present invention may contain cells, tissue and/or DNA originating from a host organism, e.g. a human patient, the intension of the invention is to test for the presence of microorganisms present within that host - not to test the host's own DNA.

The solid substrate of the invention is suitably selected from an absorbent fibrous material impregnated with one or more reagents that act to immobilize and inactivate any microorganisms present in the biological sample, or even more simply to cause immobilization of nucleic acid contained within the microorganisms. Such reagents may include detergent compounds: anionic or cationic detergents, chelating agents (e.g. EDTA), urea and/or uric acid. The solid substrate itself may incorporate an absorbent a material selected from a cellulose-based paper (e.g. blotting paper); a microfibrous membrane; a glass-fibre material; a polymeric fibre material (e.g. a nylon filter membrane); a woven fabric; and a non-woven fabric. Suitable solid substrates are described, for example, in US Patent Nos. 5496562, 5756126, 5807527, 5939259, 5972386, 5985327, 6168922, 6746841 and 6750059. In specific embodiments of the present invention the solid substrate includes filter paper treated with Whatman FTA^{®} or Whatman FTA Elute^{®} reagent, such as Whatman FTA® paper or Whatman FTA® Elute paper.

A significant advantage of the method of the present invention is that a solid substrate treated with reagent, such as Whatman FTA Elute^{®} reagent, can be used by an individual in a non-clinical setting. For instance, it is envisaged that the initial sample collection phase of the method of the invention could be carried out by an individual purchasing a kit and simply wetting the solid sold substrate with, say, a sample of urine or saliva. The solid substrate will immobilize any microorganisms present in the urine or saliva, such that infectious pathogens are safely rendered non-infectious and the sample can be transmitted to a testing location without the need for expensive handling (i.e. refrigeration, or additional chemical fixing), for instance by regular postal services. The immobilized microorganism DNA can be easily extracted from the solid substrate in the testing location, typically by using a simple heat and water elution step. The invention, thus, provides a system in which biological sample collection can even be performed at home or in the field, samples can be stored indefinitely and then tested at a later date. The benefits of this arrangement are significant as to-date most diagnostic testing is hampered by the need for sample collection to be performed in a clinical environment, which reduces uptake amongst the population as a whole.

It is envisaged that a suitable home testing kit may include a sealable chamber that encloses a testing surface. The kit may include instructions for use, which direct the user to place a biological sample - such as a drop of urine - on the testing surface. The testing surface comprises a solid substrate of the type described above. After the sample is deposited on the testing surface the sealable chamber can be closed such that it encapsulates and protects the testing surface from further interference. In the sealed state the kit remains secure from outside interference or contamination and can be despatched to a testing facility located remotely from the user's home. At the testing facility the sealable chamber can be opened (if necessary by breaking the chamber open) allowing access to the testing surface for analytical purposes according to the method of the invention.

A "nucleic acid sequence" is a single or double stranded covalently-linked sequence of nucleotides in which the 3' and 5' ends on each nucleotide are joined by phosphodiester bonds. The nucleic acid sequences are typically polynucleotides that may be made up of deoxyribonucleotide bases or ribonucleotide bases. Polynucleotides include DNA and RNA, and may be manufactured synthetically in vitro or isolated from natural sources. Sizes of nucleic acid sequences are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called "oligonucleotides". The primer sequences utilised in the present invention for the nucleic acid amplification and primer extension steps are single stranded oligonucleotides.

The term "nucleic acid amplification reaction" as used herein denotes any of a number of related enzymatic techniques that utilise a thermostable DNA polymerase to amplify a specified sequence of DNA using serial rounds of primer extension, denaturation and hybridisation. Typically, PCR is the preferred nucleic acid amplification reaction used in the method of the present invention.

The term "primer extension reaction" is intended to denote a reaction in which nucleic acid primers are designed to hybridize with a target sequence and be enzymatically extended by adding one or more nucleotides to the 3'-end of the primer. The primers hybridise at a position on a given target sequence that is immediately 5' to or a few bases upstream of the position of a polymorphism, such as a single nucleotide polymorphism. In embodiments of the invention where the primer hybridises one base upstream of a known SNP site, single-base primer-extension acts on primer-target sequence hybrids to add a single chain-terminating nucleotide, often a dideoxynucleotide. The only one of four nucleotides that will extend the primer is the one that is complementary to the sequence on the target strand. The identity of the added nucleotide is determined during the analysis phase of the method of the invention, described in more detail below.

The term "polymorphic allele" is used herein to denote any two or more alternative forms of genetic sequence occupying the same chromosomal locus and controlling the same inherited characteristic. Allelic variation arises naturally though mutation, and may result in phenotypic polymorphism within populations or may result in a conservative (non-phenotypic) polymorphism. Gene mutations typically result in an altered nucleic acid sequence. As used herein, the phenomenon of allelic polymorphism is utilised in respect of single nucleotide polymorphisms (SNPs), insertions, deletions, inversions and substitutions, all of which can occur even in genes that are highly conserved in a given species. SNPs are polymorphisms where the alleles differ by the replacement/substitution of a single nucleotide in the DNA sequence at a given position in the genome. In highly conserved genes, such as 16S rRNA in bacteria, SNPs are highly species and strain specific, thereby allowing accurate genotyping information to be obtained. Other highly conserved regions in microorganisms include the bacterial 32S rRNA gene, yeast 16S and 18S rRNA genes and viral polymerase genes. Nevertheless, it is within the remit of the skilled person to utilise bioinformatics techniques to identify SNPs in other alternative conserved regions of the genome for a given microorganism. Polymorphisms, such as those described above, may be linked to specific phenotypic traits in the organism under test. For instance, antibiotic resistance is associated with mutation and, thus, polymorphism. Nevertheless, the method of the present invention is not restricted to identification of only polymorphic positions in the genomes of the microorganisms under test. Where competitor control sequences are used for a given conserved target sequence, the term "polymorphic allele" is used loosely to denote the variation at a given part of the sequence between the wild type sequence (that being tested for) and the artificial competitor sequence. In this instance it will be appreciated that the so-called polymorphism is simply to assist in differentiation of the reaction products of primer extension on the competitor template versus the wild type template, as the respective reaction products will have differing relative molecular masses.

The present invention is based in part upon a method for reliable and high throughput testing of one or more biological samples for the presence of microorganisms in that sample. The high throughput analysis is enabled by the use of a diagnostic multiplexing panel (DMP) that is directed towards genotyping of a plurality of microorganisms that are potentially present in the biological sample. The DMP provides a combination of primers that each specifically hybridise with a highly conserved sequence in DNA that is isolated from microorganisms that may be present within a biological sample. The DMP allows for simultaneous primer extension reactions to identify if one or more of a plurality of organisms are potentially present in a single sample. The DMPs of the present invention may suitably be directed at particular therapeutic or diagnostic areas, wherein the microorganisms being tested for fall broadly within a disease area or type. In an example of the invention in use described in more detail below, a DMP is assembled directed at diagnosis of the presence of sexually transmitted infection in biological samples taken from human patients. This form of DMP can suitably test for the presence of bacterial pathogens such as Mycoplasma spp.; Chlamydia spp.; Ureaplasma spp; Neisseria spp.; Gardnerella spp.; Trichomonas spp.; Treponema spp; or the yeast Candida albicans; or viral pathogens such as: cytomegalovirus (CMV); hepatitis viruses (e.g. HAV, HBV and HCV etc.); human immunodeficiency viruses (HIV); human papilloma viruses (HPV); herpes simplex viruses (HSV); Molluscum contagiosum virus (MCV); influenza virus; Epstein-Barr virus (EBV) and varicella-zoster virus (VZV).

Other disease areas to which DMPs may bey directed include: food poisoning; tuberculosis; virally induced cancer; encephalitis; malaria; hepatitis; meningitis; leishmaniasis; African trypanosomiasis; pneumonia; plague; SARS; MRSA; rabies; anthrax; Rift valley fever; tularemia; shigella; botulism; yellow fever; Q fever; ebola; dengue fever; West Nile fever; dysentery; influenza; measles; and typhus.

It is envisaged that the invention may further enable detection of sequences that confer antibiotic sensitivity in bacterial pathogens by including such sequences in the DMP testing design. This allows speeding up the commencement of treatment of individuals found to be harbouring such pathogens by removing the additional separate step of microbiological identification of antibiotic sensitivity. Furthermore, it is envisaged that the invention may provide information about the progression of some diseases by determining the concentration of detected pathogens, which in many cases reflects the progress of the disease. Concentration may include, for example, an assessment of viral load. Quantitative information can be obtained from the primer extension phase of the reaction, for instance, by inclusion of a competitor sequence to a given target sequence, which competitor sequence contains an introduced polymorphism at a specified position in its sequence compared to the target sequence. The competitor sequence can suitably include an alternative nucleotide at the position of a known SNP but which is otherwise identical. If the competitor is supplied during the nucleic acid amplification stage at a known concentration (or copy number) then is can serve as a benchmark for quantifying concentration of the polymorphism-containing target sequence from the microorganism of interest. In a specific embodiment of the invention it is possible to provide additional competitor sequences at different concentrations (e.g. low, medium and high concentration) all with an introduced sequence variation directed at a specific site in a target sequence to enable more accurate quantification of the microorganism concentration in the original biological sample. Quantification aside, inclusion of competitor sequences also provides an internal control for all the enzymatic steps in the diagnostic method of the invention.

The DMP used in accordance with the invention is suitably provided as a plurality of appropriately plexed primers in solution. However, the DMP can also comprise primers that are immobilized on a solid surface such as in the form of a microarray. The solid surface can suitably be in the form of a silicon substrate or a glass substrate.

Resolution of the DMP reaction products following primer extension can be achieved using a number of technologies, including mass spectrometry (e.g. MALDI-TOF), electrophoresis (e.g. capillary electrophoresis), DNA microarray (e.g. Affymetrix's GeneChip™ or printed DNA arrays), via incorporation of fluorescently labelled nucleotides (e.g. Beckman Coulter's SNPstream^{®} or Applied Biosystems' SNPIex^{®}), or other labels (e.g. antigen, biotin, or a radiolabel). The preferred method for resolution of the primer extension products involves determination according to relative molecular weight, both mass spectrometry and capillary electrophoresis are favoured for this.

In one specific embodiment of the present invention, each primer comprised within the DMP varied by overall nucleotide length such that no two primers were of the same relative molecular weight either before or after the primer extension reaction. The products of the reaction are purified in order to optimise mass spectrometric analysis. After purification the products were spotted onto an appropriate element, typically a silicon chip incorporating high-density, photo-resistant array of mass spectrometry analysis sites (e.g. a SpectroCHIP®) and analysed on a matrix assisted laser desorption/ionisation-time-of-flight (MALDI-TOF) mass spectrometer (e.g. Sequenom's MassArray® Mass Spectrometer as described in US Patent Nos. 6500621, 6300076, 6258538, 5869242, 6238871, 6440705, and 6994969). The results of mass spectrometric analysis will be processed using an appropriate software package, so as to provide information on the presence or absence of primer extension products that are correlated to the presence or absence of particular specified microorganisms in the biological sample.

The invention is further illustrated by the following non-limiting example.

### EXAMPLE

The present inventors have developed a cost effective, robust and highly accurate test, which has the potential to determine any number of infections in a single sample. The test comprises two parts - a simple home collection kit (utilising Whatman FTA Elute^{®} paper as the sample carrying substrate) and a novel sexually transmitted infection Multiplex Panel (STIMP) as the Diagnostic Multiplex Panel (DMP) which can determine whether any given individual is infected with one or several sexually transmitted bacterial, viral, protozoan and/or fungal pathogens using DNA based technology.

The present example demonstrates that:
- Whatman FTA Elute^{®} paper is an adequate carrier of bacterial, fungal and protozoan pathogens derived from a human urine sample.
- compounds present within Whatman FTA Elute^{®} paper or urine do not interfere with down stream enzymatic testing processes.
- the novel STIMP functions as a DMP and is able to detect individual pathogens from a pathogen mixture.

### MATERIALS & METHODS

### • CLINICAL SAMPLES

A total of 44 samples were obtained from patients attending a private GUM (Genito-Urinary Medicine) clinic located in the Ukraine.

Patients in the experimental group were asked to provide a first void urine sample (approximately 30ml-50ml) in a sterile collection pot upon attending the consultation. All samples were provided with informed patient consent and all ethical requirements and regulations (including the method of sample collection) were met and carried out accordingly as stipulated by the Ukrainian Department of Health.

Each urine sample was transferred onto a Whatman FTA Elute^{®} (Whatman plc, Brentford, UK) sample card by dipping a sterile foam tipped applicator (Puritan^{®}, Maine, USA) once into the urine sample pot and then blotting four times in four separate areas on the card.

After sample transfer each individual Whatman FTA Elute^{®} card was dried at room temperature until completely dry. To prevent cross contamination dry sample cards were placed individually inside a self-seal polythene bag and further stored at room temperature.

Batch 1 and 2 samples (31 and 13 separate samples respectively) were then shipped in two consignments (via Federal Express^{®} courier service) to the testing location at a laboratory in Germany. Batch 1 sample collection was undertaken during the period 12 December - 26 December 2006 inclusive. Batch 2 sample collection was undertaken during the period 5 January - 12 January 2007 inclusive.

All samples were analysed in parallel by an independent local laboratory (Kiev, Ukraine) which specialises in Sexually Transmitted Infection testing utilising conventional DNA based detection techniques as recommended by the Ukrainian Department of Health.

All samples were tested for presence of the following microorganisms:
*Candida albicans*
*Chlamydia trachomatis*
*Gardnerella vaginalis*
*Mycoplasma genltalium*
*Mycoplasma hominis*
*Neisseria gonorrhoeae*
*Trichomonas vaginalis*
*Treponema pallidum*
*Ureaplasma urealyticum*

The results obtained from the local testing laboratory (designated as the 'clinic') were then compared using the results obtained utilising the STIMP/DMP method (designated as the 'lab').

### • EXPERIMENTAL PROCEDURES

### DNA EXTRACTION

To account for differences in DNA concentration six 6mm sample disks from each Whatman FTA Elute^{®} sample card were excised using a hand held punching device in the following sequence - 1 x circle per 2 ml tube, 2 X circles per 2 ml tube, 3 x circles per 2 ml tube. The punching device was cleaned after excising of each sample card by punching through clean filter paper three times followed by punching through filter paper soaked with 70% EtOH a further three times. To account for cross contamination a clean FTA Elute^{®} card was then punched once and DNA extracted as below (tube number 4).

DNA extraction was performed using Whatman's FTA Elute card^{®} DNA extraction protocol in our modification in order to account for a 6mm sample disk punch as opposed to the recommended 3mm sample disk punch. One, two and three 6mm sample disks or Excised Paper Fragments (EPF) were placed into separate 2ml round bottom Eppendorf tubes to which 0.7ml, 1.4ml and 2.1ml of ddH₂O was added respectively.

Each sample was vortexed for 5 seconds three times and the sample disks transferred into separate clean 0.5ml Eppendorf tubes.

50µl, 80µl and 100µl of ddH₂O were added to each tube containing one, two and three sample disks respectively after which the tubes were incubated at 95C for 30 minutes. After incubation the samples were spun at 12000g for 2 minutes and stored at +4°C. For PCR amplification 1µl of each sample was used directly or as a 1:1 dilution with ddH₂O

### POSITIVE CONTROLS

The following cell lines obtained from the National Collection of Type Cultures (NCTC) were used as positive controls.

### Cell line No. Species

- NC12700: *N. gonorrhoeae*
- NC11148: *N. gonorrhoeae*
- NC08448: *N. gonorrhoeae*
- NC10177: *U. urealyticum*
- NC10111: *M. hominis*
- NC10915: *G. vaginalis*
- NCPF3179: *C. albicans*
- NC10195: *M. genitalium*

The cells were diluted with 500µl of fresh urine and then 50µl from each sample was taken to make positive controls as follows.

### Positive control No. 1

- NC12700: *N. gonorrhoeae*
- NC10177: *U. urealyticum*
- NC10111: *M. hominis*
- NC10915: *G. vaginalis*
- NCPF3179: *C. albicans*
- NC10195: *M. genitalium*

### Positive control No. 2

- NC11148: *N. gonorrhoeae*
- NC10177: *U. urealyticum*
- NC10111: *M. hominis*
- NC10915: *G. vaginalis*
- NCPF3179: *C. albicans*
- NC10195: *M. genitalium*

### Positive control No. 3

- NC08448: *N. gonorrhoeae*
- NC10177: *U. urealyticum*
- NC10111: *M. hominis*
- NC10915: *G. vaginalis*
- NCPF3179: *C. albicans*
- NC10195: *M. genitalium*

The positive controls and the fresh urine sample used for diluting the cells were pipetted onto individual FTA Elute^{®} cards as recommended by Whatman (50µl per ca 1 cm²). After application of the samples the cards were dried at 60°C for one hour and DNA extracted as above.

In addition an aliquot of each positive control and the fresh urine sample (used for diluting the cells) were used both directly and at 1:5 dilution in ddH₂O for PCR amplification.

At the time of the experiment we were unable to obtain positive controls for C. *trachomatis, T. vaginalis or T. pallidum.*

### ASSAY DESIGN

The DMP assay design is based on DNA sequences highly conserved between different strains of each species of interest. To account for high variability, three different areas in the conserved regions were used to design three assays for each pathogen. For the all pathogens the conserved region chosen was the 16S rRNA gene, except for *C. albicans* where the conserved region chosen was the 18S rRNA gene (see Fig. 1).

For *U. urealyticum* two different conserved areas were used to design six assays. The exact sequences for PCR and primer extension (PE) primers are shown in Table 1.

The assay identifies the presence of the following bacterial, fungal and protozoan species in the sample.
*C. albicans*
*C. trachomatis*
*G. vaginalis*
*M. genitalium*
*M. hominis*
*N. gonorrhoeae*
*T. vaginalis*
*T. pallidum*
*U. urealyticum*

Two types of assays were performed. The first type included control competitor sequences for each target, the second did not contain any competitors. The role of the competitor is to serve as the internal positive control for PCR, PE and other enzymatic reactions as well as chip spotting. In a sample with a competitor it would be expected to see at least a signal for the competitor even if the sample did not contain the corresponding DNA target from the pathogen. The competitors were designed to be identical to the target DNA sequence with a known single nucleotide difference at the site of the SNP of interest, the sequences of the competitors are shown in Table 1. The competitors were added at an approximate amount of 30 copies per target per PCR amplification. In the samples analysed without competitors a signal was expected only when the target DNA from the pathogen was present in the sample. Targets from the same multiplex serve as internal positive controls for each other, however if the signal is absent for all targets amplified together it is impossible to conclude • whether the sample does not contain any target pathogen DNA or that one or several enzymatic reactions failed.

### PCR AMPLIFICATION

PCR amplification was performed in 5µl reaction volume containing 1.25x HotStar^{®} PCR Buffer, 1.625mM MgCl₂, 0.04mM of each dNTP, 0.1 µM of each primer and 0.1 U of HotStarTaq^{®}. Cycling parameters were as follows:
- 95°C: 15 min
- 94°C: 20 sec
- 56°C: 30 sec
- 72°C: 1 min
For 45 cycles
- 72°C: 3 min
- 4°C: 5 min
- 15°C: Forever

Amplification was performed on an MJR Tetrad Thermo Cycler.

### SHRIMP ALKALINE PHOSPHATE (SAP) TREATMENT

After PCR amplification the following was added to each tube: 1.53µl of H₂O, 0.17µl of TS buffer and 0.30µl of SAP. The reaction was performed using the following parameters:
- 37°C: 20 min
- 85°C: 5 min
- 4°C: Forever

The reaction was performed on an MJR Tetrad Thermo Cycler.

### PRIMER EXTENSION REACTION

Primer extension was carried out according to the iPLEX^{™} protocol (Sequenom, Inc., San Diego, CA, USA). After incubation 2µl of the iPLEX primer extension cocktail containing 1x iPLEX buffer, 1x iPLEX termination mix, 0.625µM of each extension primer and 1x iPLEX enzyme was added to each tube. iPLEX reaction was performed using the following parameters:
- 94°C: 30 sec
- 94°C: 5 sec
- 52°C: 5 sec
- 80°C: 5 sec
Go to step 3 X 5 times
Go to step 2 X 40 times
- 72°C: 3 min
- 4°C: Forever

The reaction was performed on an MJR Tetrad Thermo Cycler.

### DESALTING

16µl of H₂0 and 6mg of SpectroCLEAN^{®} resin was added to the above mixture and rotated on a circular shaker for 30 minutes and then centrifuged for 5 min at 3,000 g to precipitate the resin.

### SAMPLE SPOTTING

9 nanolitres of each sample were spotted onto a 384 SpectroCHIP^{®} using a MassARRAY^{®} Nanospotter robot as per the manufacturers instructions. Each chip was then read on a MALDI-TOF mass spectrometer, a MassARRAY^{®} Compact Analyser, and data collected using Typer v3.3 (or above) and stored in the database.

### GENOTYPE SCORING & DETERMINATION OF INFECTION STATUS OF SAMPLE

For each assay (DNA target) a specific competitor was designed to differ from the target DNA by an artificially introduced single nucleotide polymorphism (SNP). The latter was designated as the MUT (Mutant) genotype by the genotyping software. The pathogenic SNP was designated as C (Control) genotype. When both competitor and the target DNA were present in the reaction a heterozygous genotype designated as either C.MUT or MUT.C was scored by the software. The order of the alleles in the heterozygous genotype reflects the relative proportion of corresponding DNA in the reaction.

The sample was considered to be infected when at least two out of the three assays indicated the presence of pathogenic DNA in a single DNA sample. As three different DNA samples were obtained from each patient sample card it was expected that all three samples should show identical results (NB. because each sample potentially contained different copy numbers of a pathogen, DNA samples derived from two and three sample disks were expected to produce more reliable results due to increased amounts of pathogenic DNA in comparison with a DNA sample extracted from a single sample disk). Occasionally only one or two assays indicated the presence of pathogenic DNA in a patients sample while the majority of assays for this pathogen on all DNA samples were negative. These results were thought to be artefacts (most probably due to cross contamination) however the samples were scored as positive and indicated by the presence of a 'x' cross next to the result within the summary results table (Table 2).

### • RESULTS & DISCUSSION

### Whatman FTA Elute^{®} paper

To investigate whether any compounds present within Whatman FTA Elute^{®} paper or urine can interfere with down stream enzymatic processes a series of mock samples containing an aliquot of known good quality DNA was diluted 1:1 with eluant obtained after extracting one, two and three sample disks or with fresh urine. The samples were then used for PCR and other downstream enzymatic reactions. For all the samples good quality PCR products and mass spectrum was obtained (data not presented). This indicates that the chosen extraction method and washing protocol are adequate for MALDI-TOF analysis and also show that substances in human urine do not significantly affect the reliability of the DMP analysis.

To determine whether Whatman FTA Elute^{®} paper is able to capture and preserve pathogenic DNA derived from a urine sample several DNA samples (extracted from the cards) belonging to the patients identified as positive by the local laboratory analysis (clinic) were amplified in a 50µl reaction using the primers from the STIMP/DMP and the products separated on 2% agarose gel in 1xTBE buffer (see Fig. 1). In all cases a good signal for the correct size amplicons was observed indicating the presence of pathogenic DNA on Whatman FTA Elute^{®} paper in amounts sufficient for reliable PCR amplification.

The same experiment was performed using positive control urine samples and similar results were observed (data not presented).

These results confirm that cellulose based products such as Whatman FTA Elute^{®} paper can be used as a suitable solid substrate both collect and transport patient samples and show no visible time dependant deterioration. Nevertheless, it is envisaged that crude urine samples as well as biological samples immobilised on other types of substrate can also be used for testing utilising the present STIMP/DMP method.

### STIMP/DMP METHODOLOGY

All the samples were tested in reactions with or without competitor control sequences. The experiment in which competitors were included produced signals for competitor DNA, in most cases including positive controls.

In this section the results obtained from the experiments when competitors were not included in the reaction are discussed.

The results of the experiments are presented in Table 2.

### POSITIVE CONTROLS

To confirm whether the STIMP/DMP method can be used for detecting individual pathogens from a pathogen mixture three positive controls obtained by mixing sexually transmitted pathogens obtained from the NCTC were created using urine as a medium (see above). The positive control samples were analysed both as urine samples and as Whatman FTA Elute^{®} paper samples (Table 3). In all cases the presence of each pathogen within the sample was detected independent of its nature. Positive controls differed by the strain of *N. gonorrhoeae* present in them. In all the assays for this species the STIMP/DMP method was able to detect the presence of *N. gonorrhoeae* independent of the strain type. This shows that the regions of DNA chosen for developing the *N. gonorrhoeae* assays do not show strain-dependant specificity and can be used to detect the presence of any known *N. gonorrhoeae* species in the sample.

### CLINICAL SAMPLES

When analysing the clinical samples the STIMP/DMP lab results confirmed the results obtained and analysed independently from the clinic in all cases (Table 2). Several samples were also identified as positives for infections not detected by the testing laboratory. However, because the pathogen was not detected by all assays and because only one (rarely two) DNA samples had a positive signal these results should be treated as artefacts of this pilot study. In most cases this occurred when the patient whose sample was on the previously punched sample card was positive for a particular infection (e.g. patient 2 & 3 - *U. urealyticum*, patients 11 & 12 - *M. genitalium* etc). The most likely explanation for these artefacts is the carrying over of DNA from the previous sample. This is supported by the fact that contamination control samples (clean Whatman FTA Elute^{®} card) used in between patient samples were also found to be positive for the same infections. Clearly this demonstrates the high sensitivity of the DMP of the invention and future experimental procedures will be optimised to avoid cross contamination.

Three different DNA samples were collected from each patient sample card. The samples contained different copy number of pathogenic DNA (when present). In the majority of cases it was possible to identify the infection in DNA samples from positive patients extracted from two or more sample disks.

For most DNA samples from positive patients all three assays identified infection. In a very small number of instances one of the three assays did not produce good quality signal and was flagged by the genotyping software.

It is also possible to determine the copy number of target DNA present in the reaction by titrating the amount of competitor or including three different competitors at known initial concentrations in the same reaction. The latter approach is recommended for pathogens whose level of infectious load is important from a clinical perspective, for example in individuals infected with HIV or *G. vaginalis*.

**Table 1**

| Multiplex Reaction 1 | | | | | | | | | |
|---|---|---|---|---|---|---|---|---|---|
| PATHOGEN | ASSAY NAME | PCR PRIMER 1 (5'-3') | PCR PRIMER 2 (5'-3') | EXTENSION PRIMER (5'-3') | COMPETITOR (5'-3') | PATHOGEN SNP | MW OF THE PATHOGEN | COMPETITOR SNP | MW OF T HE COMPETITOR |
| M. hominis | MH3 | ACGTTGGATGCATACTAC TCAGGCGGATCA (SEQ ID NO = 1) | ACGTTGGATGCGATGAT CATTAGTCGGTGG (SEQ ID NO = 2) | TCACTGACGCAGC TAA (SEQ ID NO = 31) | CATACTACTCAGGCGGATCATTTAATGCaTTAGCTGCGTCAGTGATTCTCCACCGACTAATGATCAT CG (SEQ ID NO = 91) | C | 5097.4 | T | 5177.3 |
| M. hominis | MH1 | ACGTTGGATGGCTTACCT CTATCTAACTC (SEQ ID NO = 3) | ACGTTGGATGAAGTCTG GAGTTAAATCCCG (SEQ ID NO = 4) | CGGCTCGCTTTGG ATA (SEQ ID NO = 32) | GCTTACCTCTATCTAACTCTAGTTTGCTAaTATCCAAAGCGAGCCGGGGTTGAGCCCCGGGATTTAA CTCCAGACTT (SEQ ID NO = 92) | C | 5135.4 | T | 5215.3 |
| u.realiticum | UU2/1 | ACGTTGGATGAACCCAAC ATCTCACGACAC (SEQ ID NO = 5) | ACGTTGGATGAACAATA TGACAGGTGGTGC (SEQ ID NO = 6) | GGTGGTGCATGGT TGT (SEQ ID NO = 33) | AACCCAACATCTCACGACACGAGCTGACaACAACCATGCACCACCTGTCATATTGTT (SEQ ID NO = 93) | C | 5246.4 | T | 5326.3 |
| C. trachomatis | CT2 | ACGTTGGATGTTCGCCAC TGGTGTTCTTCC (SEQ ID NO = 7) | ACGTTGGATGAGATGGA GAAAAGGGAATT (SEQ ID NO = 8) | CGTGTAGCGGTGA AATG (SEQ ID NO = 34) | TTCGCCACTGGTGTTCTTCCACATATCTACaCATTTCACCGCTACACGTGNAATTCCCTTTTCTCCA TCT (SEQ ID NO = 94) | C | 5537.6 | T | 5617.5 |
| G. vaginalis | CV2 | ACGTTGGATGTTGGAGCA TCCAGCATTACC (SEQ ID NO = 9) | ACGTTGGATGAGTAATG CGTGACCAACCTG (SEQ ID NO = 10) | CCCCATGCTCCAG AATAG (SEQ ID NO = 35) | TTGGAGCATCCAGCATTACCACCCGTTTCCAAGAaCTATTCTGGAGCATGGGGCAGGTTGGTCACGC ATTACT (SEQ ID NO = 95) | C | 5675.7 | T | 5755.6 |
| T. pallidum | TP1 | ACGTTGGATGGTCCGCCA CTCTAGAGAAAC (SEQ ID NO = 11) | ACGTTGGATGGCGTTTT AAGCATGCAAGTC (SEQ ID NO = 12) | AAGCATGCAAGTC GAACGG (SEQ ID NO = 36) | GTCCGCCACTCTAGAGAAACGAAAATTCGCTTCCTTaCCGTTCGACTTGCATGCTTAAAACGC (SEQ ID NO = 96) | C | 6118 | T | 6197.9 |
| T. pallidum | TP3 | ACGTTGGATGTCAATCCG GACTACGATTGC (SEQ ID NO = 13) | ACGTTGGATGACAATGG TTGCTACAGAGCG (SEQ ID NO = 14) | GTTGTGAAGTGGA GCAAAC (SEQ ID NO = 37) | TCAATCCGGACTACGATTGCCTTTTTGCaGTTTGCTCCACTTCACAACCTCGCATCGCTCTGTAGCA ACCATTGT (SEQ ID NO = 97) | C | 6164.1 | T | 6244 |
| U. urealiticum | UU1/1 | ACGTTGGATGTACGTGTT ACTCACCCGTTC (SEQ ID NO = 15) | ACGTTGGATGTGGCCGGC ATGCCTAATACAT (SEQ ID NO = 16) | CCTAATACATGCA AATCGAA (SEQ ID NO = 38) | TACGTGTTACTCACCCGTTCACCACTAAGCCTNAAAGGCTTCaTTCGATTTGCATGTATTAGGCATG CCGCCA (SEQ ID NO = 98) | C | 6325.2 | T | 6405.1 |
| G. vaginalis | GV3 | ACGTTGGATGACAAGCTG ATAGGACGCGAC (SEQ ID NO = 17) | ACGTTGGATGTTGACGC ATGTCTTGTTGGG ((SEQ ID NO = 18) | GTTGGGAAAGTGT TTAGTGG (SEQ ID NO = 39) | ACAAGCTGATAGGACGCGACCCCATCCCATaCCACTAAACACTTTCCCAACAAGACATGCGTCAA (SEQ ID NO = 99) | C | 6530.3 | T | 6610.2 |
| M. genitalium | MG2 | ACGTTGGATGTTGCTCCC CACACTTTCAAG (SEQ ID NO = 19) | ACGTTGGATGTGGCCGAA GGCGAAAACTTAG (SEQ ID NO = 20) | AGGCGAAAACTTA GGCCATTA (SEQ ID NO = 40) | TTGCTCCCCACACTTTCAAGCCTAAGCGTCAaTAATGGCCTAAGTTTTCGCCTTCGCCA (SEQ ID NO = 100) | C | 6710.4 | T | 6790.3 |
| T. vaginalis | TV1 | ACGTTGGATGATGAGGTC AACTTTTCCTCC (SEQ ID NO = 21) | ACGTTGGATGCTCTGGT GCTAATACATGCG (SEQ ID NO = 22) | CTAATACATGCGA TTGTTTCTC (SEQ ID NO = 41) | ATGAGGTCAACTTTTCCTCCATAATTCACATCTaGAGAAACAATCGCATGTATTAGCACCAGAG (SEQ ID NO = 101) | C | 6922.5 | T | 7002.5 |
| U.urealiticum | UU1/3 | ACGTTGGATGAAATTCCC TACTGCTGCCTC (SEQ ID NO = 23) | ACGTTGGATGGTACTGA GAGGTAGAACAGC (SEQ ID NO = 24) | AGGTAGAACAGCC ACAATGGGA (SEQ ID NO = 42) | AAATTCCCTACTGCTGCCTCCCGTAGGAGTATGGGCCGTGTCTCAaTCCCATTGTGGCTGTTCTACC TCTCAGTAC (SEQ ID NO = 102) | C | 7073.7 | T | 7153.6 |
| C. albicans | CA3 | ACGTTGGATGTTATTCCA GCTCCAAAAGCG (SEQ ID NO = 25) | ACGTTGGATGAAGCCCA AGGTTCAACTACG (SEQ ID NO = 26) | AGGTTCAACTACG AGCTTTTTAA (SEQ ID NO = 43) | TTATTCCAGCTCCAAAAGCGTATATTAAAGTTGTTGCAaTTAAAAAGCTCGTAGTTGAACCTTGGGC TT (SEQ ID NO = 103) | C | 7284.8 | T | 7364.7 |
| T. vaginalis | TV2 | ACGTTGGATGTCATGAGA GAGAAGCTGAGG (SEQ ID NO = 27) | ACGTTGGATGCGCCCCTT GATCGACAGAAAC (SEQ ID NO = 28) | CCCTTGATCGACA GAAACCCTCTA (SEQ ID NO = 44) | CGCCCTTGATCGACAGAAACCCTCTAcGTAGACGCCTTCGCCTCAGCTTCTCTCTCATGA (SEQ ID NO = 104) | G | 7488.9 | C | 7488.9 |
| U. urealiticum | UU2/3 | ACGTTGGATGCGATCCTA CCCTAGACGCAT (SEQ ID NO = 29) | ACGTTGGATGCGTCAAA CTATGGGAGCTGG (SEQ ID NO = 30) | TCAAACTATGGGA GCTGGTAATAT (SEQ ID NO = 45) | CGATCCTACCCTAGACGCATNCCTCCAAAAGGTTAGCTTTGNCGGTTTTAaATATTACCAGCTCCCA TAGTTTGACG (SEQ ID NO = 105) | C | 7663 | T | 7742.9 |

| Multiplex reaction 2 | | | | | | | | | |
|---|---|---|---|---|---|---|---|---|---|
| PATHOGEN | ASSAY NAME | PCR PRIMER 1 (5'-3') | PCR PRIMER 2 (5'-3') | EXTENSION PRIMER (5'-3') | COMPETITOR (5'-3') | PATHOGEN SNP | MW OF THE PATHOGEN ALLELE, D | COMPETITOR SNP | MW OF THE COMPETITOR ALLELE, D |
| N. gonorhoea | NG2 | ACGTTGGATGTAGCGTAG CTAACGCGTGAA (SEQ ID NO = 46) | ACGTTGGATGGAGTTTT AATCTTGCGACCG (SEQ ID NO = 47) | CGACCGTACTCCC CAG (SEQ ID NO = 76) | GAGTTTTAATCTTGCGACCGTACTCCCCAGcCGGTCAATTTCACGCGTTAGCTACGCTA (SEQ ID NO = 106) | G | 5074.3 | C | 5034.3 |
| C. trachomatis | CT3 | ACGTTGGATGTGTGTACA AGGCCCGGGAAC (SEQ ID NO = 48) | ACGTTGGATGTGCTAGT AATGGCGTGTCAG (SEQ ID NO = 49) | CGTGTCAGCCATA ACG (SEQ ID NO = 77) | TGTGTACAAGGCCCGGGAACGTATTCACGaCGTTATGGCTGACACGCCATTACTAGCA (SEQ ID NO = 107) | C | 5113.4 | T | 5193.3 |
| M, hominis | MH2 | ACGTTGGATGCAGCGTCA GTATAGACCCAG (SEQ ID NO = 50) | ACGTTGGATGTGAAGCG GTGAAATGCGTAG (SEQ ID NO = 51) | AACACCAAAGGCG AAGG (SEQ ID NO = 78) | CAGCGTCAGTATAGACCCAGTAAGCTaCCTTCGCCTTTGGTGTTCTTCCATATATCTACGCATTTCA CCGCTTCA (SEQ ID NO = 108) | C | 5493.6 | T | 5573.5 |
| C. albicans | CAT | ACGTTGGATGGAAAGCAT TTACCAAGGACG (SEQ ID NO = 52) | ACGTTGGATGACGGTAT CTGATCATCTTCG (SEQ ID NO = 53) | TCATCTTCGATCC CCTAA (SEQ ID NO = 79) | GAAAGCATTTACCAAGGACGTTTTCATTAATCAAGAACGAAAaTTAGGGGATCGAAGATGATCAGAT ACCGT (SEQ ID NO = 109) | C | 5616.7 | T | 5696.6 |
| M. genitallum | MG1 | ACGTTGGATGGCTGCTTA ACAGTTGTATGC (SEQ ID NO = 54) | ACGTTGGATGCAAAACT CCCTACCACACTC (SEQ ID NO ≈ 55) | CCCTACCACACTC TAGACT (SEQ ID NO = 80) | CAAAACTCCCTACCACACTCTAGACTcATAGTTTCCAATGCATACAACTGTTAAGCAGC (SEQ ID NO = 110) | C | 5939.9 | G | 5899.9 |
| N. gonorhoea | NG3 | ACGTTGGATGTGGGTGAT GATTGTATCGAC (SEQ ID NO = 56) | ACGTTGGATGTGTTGTG TCTTAATCCTGCC (SEQ ID NO = 57) | TCCTGCCTTTTGT GTTTCA (SEQ ID NO = 81) | TGTTGTGTCTTAATCCTGCCTTTTGTGTTTCAcGATTAAGTCGATACAATCATCATCACCCA (SEQ ID NO = 111) | C | 6013.9 | G | 5973.9 |
| U. urealiticum | UU1/2 | ACGTTGGATGGAGCTAAT ACCGAATAATAAC (SEQ ID NO = 58) | ACGTTGGATGCTCATCC AAAAGCGTCGC (SEQ ID NO = 59) | CGACTTTCTACAT CTTCTCAT (SEQ ID NO = 82) | CTCATCCAAAAGCGTCGCNNNNAANNGCGACTTTCTACATCTTCTCATcCGATATTGATGTTATTAT TCGGTATTAGCTC (SEQ ID NO = 112) | C | 6569.3 | G | 6529.3 |
| C. albicans | CA2 | ACGTTGGATGATTAGAGT GTTCAAAGCAG (SEQ ID NO = 60) | ACGTTGGATGAGAACCA TAACGTCCTATTC (SEQ ID NO = 61) | TTCCATGCTAATA TATTCGAG (SEQ ID NO = 83) | ATTAGAGTGTTCAAAGCAGGCCTTTaCTCGAATATATTAGCATGGAATAATAGAATAGGACGTTATG GTTCT (SEQ ID NO = 113) | C | 6642.4 | T | 6722.3 |
| U. urealiticum | UU2/2 | ACGTTGGATGCGTGCTAC AATGGCTAATAC (SEQ ID NO = 62) | ACGTTGGATGCTATCCG AACTGAGACTAAC (SEQ ID NO = 63) | TGAGACTAACTTT TTCTGTTTC (SEQ ID NO = 84) | CTATCCGAACTGAGACTAACTTTTTCTGTTTCcCTTCATCTTACGATTTTGCAGCAGTTTGTATTAG CCATTGTAGCACG (SEQ ID NO = 114) | G | 6968.6 | C | 6928.5 |
| M. genitalium | MG3 | ACGTTGGATGCTATCGTT TACGGTGTGGAC (SEQ ID NO = 64) | ACGTTGGATGCTTAGGC TTGAAAGTGTGGG (SEQ ID NO = 65) | GGGAGCAAATAGG ATTAGATAC (SEQ ID NO = 85) | CTATCGTTTACGGTGTGGACTACTAGaGTATCTAATCCTATTTGCTCCCCACACTTTCAAGCCTAAG (SEQ ID NO = 115) | C | 7103.7 | T | 7183.6 |
| G. vaginalis | GV1 | ACGTTGGATGTGGCGAAC GGGTGAGTAATG (SEQ ID NO = 66) | ACGTTGGATGAGAGCTA TTCTGGAGCATGG (SEQ ID NO = 67) | TATTCTGGAGCAT GGGGCAGGTT (SEQ ID NO = 86) | AGAGCTATTCTGGAGCATGGGGCAGGTTcGTCACGCATTACTCACCCGTTCGCCA (SEQ ID NO = 116) | G | 7437.9 | C | 7397.8 |
| T. vaginalis | TV3 | ACGTTGGATGATTCCTGG TTCATGACGCTG (SEQ ID NO = 68) | ACGTTGGATGGAGGGTG CGCTACTCTTATA (SEQ ID NO = 69) | GTGCGCTACTCTT ATAATCCCTAA (SEQ ID NO = 87) | ATTCCTGGTTCATGACGCTGATTACAAACGTCAATCCCAACTACaTTAGGGATTATAAGAGTAGCGC ACCCTC (SEQ ID NO = 117) | C | 7509.9 | T | 7589.8 |
| N. gonorhoea NG1 | NG1 | ACGTTGGATGAAGTCGGA CGGCAGCACAG (SEQ ID NO = 70) | ACGTTGGATGGGTACGT TCCGATATGTTAC (SEQ ID NO = 71) | GGTACGTTCCGAT ATGTTACTCACCC (SEQ ID NO = 88) | GGTACGTTCCGATATGTTACTCACCCcTTCGCCACTCGCCACCCNAGAAGCAAGCTTCNCTGTGCTG CCGTCCGACTT (SEQ ID NO = 118) | G | 8184.3 | C | 8144.3 |
| C. trachomatis | CTI | ACGTTGGATGCCCTTCCG CCACTAAACAAT (SEQ ID NO = 72) | ACGTTGGATGATTGAAC GCTGGCGGCGTG (SEQ ID NO = 73) | GCGTGGATGAGGC ATG (SEQ ID NO = 89) | CCCTTCCGCCACTAAACAATNNNCGAANCAATTGNTCCGTTCGACTTaCATGCCTCATCCACGCCGC CAGCGTTCAAT (SEQ ID NO = 119) | C | 5249.4 | T | 5329.4 |
| T. pallidum | TP2 | ACGTTGGATGTCAATCAT CGGCCAGAAACC (SEQ ID NO = 74) | ACGTTGGATGTGTAGGG GTGGAATCTGTAG (SEQ ID NO = 75) | TGTAGATATTTGG AAGAACAC (SEQ ID NO = 90) | TCAATCATCGGCCAGAAACCCGCCTTCGCCACCaGTGTTCTTCCAAATATCTACAGATTCCACCCCT ACA (SEQ ID NO = 120) | C | 6740.4 | T | 6820.4 |

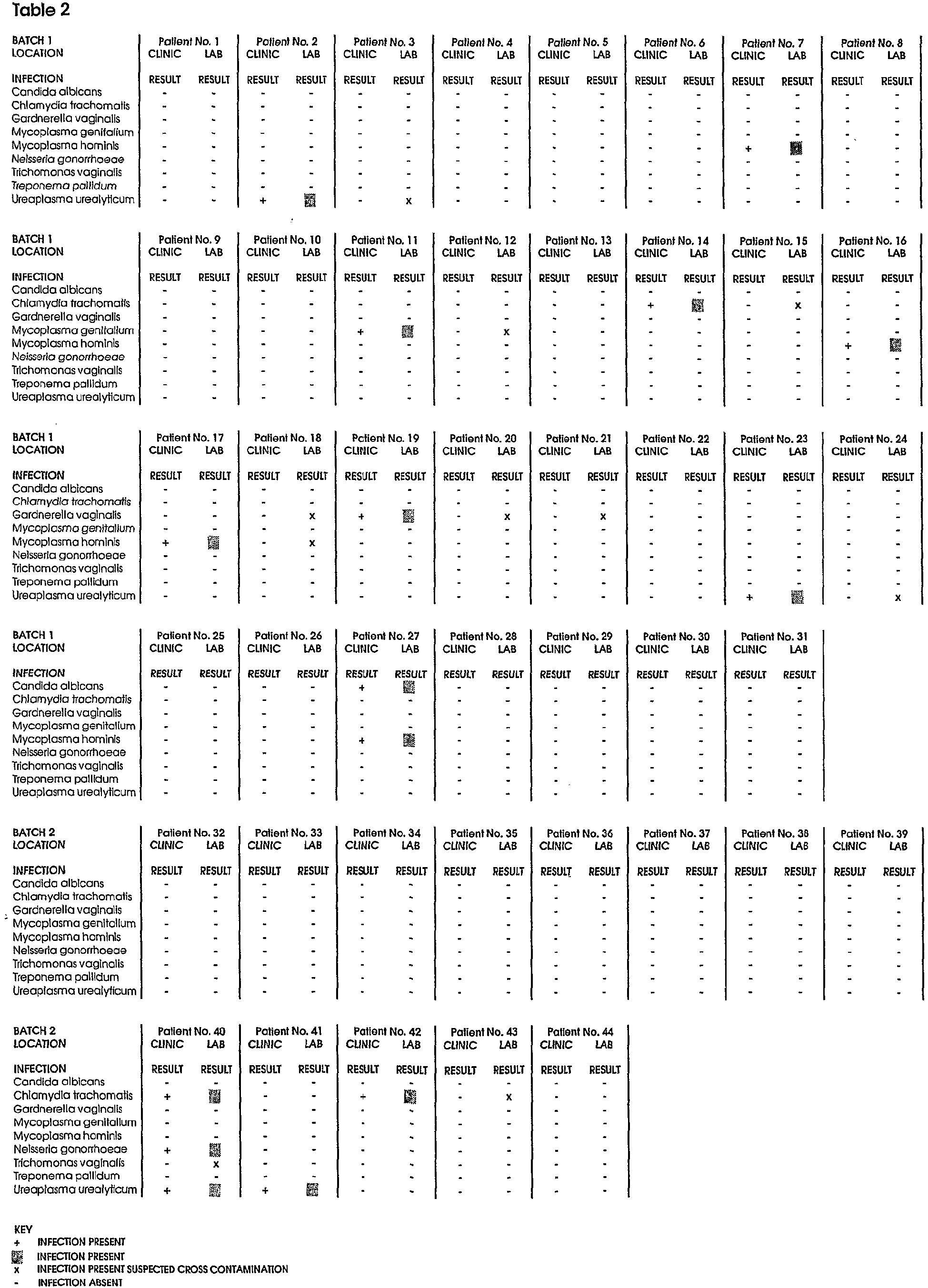

**Table 3 - Positive Control data**

| | | | Whatman FTA Elute Card | | | | | URINE | | |
|---|---|---|---|---|---|---|---|---|---|---|
| PATHOGEN | NCTC No. | | PC 1 | PC 2 | PC 3 | CONTROL | | PC 1 | PC 2 | PC 3 |
| | | | | | | | | | | |
| Neisseria gonorrhoeae | NC12700 | | + | N/A | N/A | - | | + | N/A | N/A |
| Neisseria gonorrhoeae | NC11148 | | N/A | + | N/A | - | | N/A | + | N/A |
| Neisseria gonorrhoeae | NC08448 | | N/A | N/A | + | - | | N/A | N/A | + |
| Ureaplasma urealyticum | NC10177 | | + | + | + | - | | + | + | + |
| Mycoplasma hominis | NC10111 | | + | + | + | - | | + | + | + |
| Gardnerella vaginalis | NC10915 | | + | + | + | - | | + | + | + |
| Candida albicans | NCPF3179 | | + | + | + | - | | + | + | + |
| Mycoplasma genitalium | NC10195 | | + | + | + | - | | + | + | + |

Although particular embodiments of the invention have been disclosed herein in detail, this has been done by way of example and for the purposes of illustration only. The aforementioned embodiments are not intended to be limiting with respect to the scope of the appended claims, which follow. It is contemplated by the inventors that various substitutions, alterations, and modifications may be made to the invention without departing from the scope of the invention as defined by the claims.

## Claims

1. A method for determining whether two or more species of specified microorganisms are present within a biological sample that potentially comprises the microorganisms comprising:
(a) immobilizing the biological sample on and/or within a solid substrate at a first location;
(b) transferring the immobilized biological sample to at least a second location and performing a extraction step on the solid substrate so as to extract any microorganism DNA immobilized on and/or within the solid substrate;
(c) performing a nucleic acid amplification step on microorganism DNA extracted in step (b), wherein the amplification step comprises a plurality of amplification primers that are directed towards amplification of a plurality of highly conserved sequences from the two or more specific microorganisms, and wherein amplified sequences are designated as target sequences;
(d) combining the target sequences with a plurality of primer sequences comprised within a diagnostic multiplexing panel (DMP), wherein each primer sequence facilitates genotyping of the target sequence;
(e) performing a primer extension reaction on the combination of target sequences and the DMP present in (d), thereby producing a DMP reaction product; and
(f) analysing the reaction product so as to determine genotype of any target sequences that are present and correlating the genotype of the target sequences in the reaction product with the identification of specified microorganisms present in the biological sample;
wherein the specified microorganisms are selected from one or more of the group consisting of: Mycoplasma spp., Chlamydia spp., Ureaplasma spp., Neisseria spp., Gardnerella spp., Trichomonas spp., and Treponema spp., and Candida albicans;
and wherein the DMP comprises one or more primer sequences selected from SEQ ID NOs: 31-45, and/or one or more primer sequences selected from SEQ ID NOs: 76-90.

2. The method of claim 1, wherein the first location is remote from the second location.

3. The method of claim 1 or claim, 2, wherein the biological sample comprises at least one of the group consisting of: urine; saliva; blood; sputum; semen; faeces; a nasal swab; tears; a vaginal swab; a rectal swab; a cervical smear; a tissue biopsy; and a urethral swab.

4. The method of any previous claim, wherein the solid substrate comprises an absorbent fibrous material impregnated with one or more reagents that act to immobilize and inactivate any microorganisms present in the biological sample.

5. The method of claim, 4, wherein the solid substrate comprises a material selected from a cellulose-based paper; a microfibrous membrane; a glass-fibre material; a polymeric fibre material; a woven fabric; and a non-woven fabric; and/or wherein the solid substrate comprises Whatman FTA^{®} or Whatman FTA^{®} Elute reagent or Whatman FTA^{®} Elute paper.

6. The method of any previous claim, wherein the DMP is directed towards identification of alleles from a combination of microorganisms potentially present in the biological sample, wherein the combination includes bacteria, fungi and viruses.

7. The method of claim, 6, wherein the viruses are selected from the group consisting of: cytomegalovirus (CMV); hepatitis A virus (HAV); hepatitis B virus (HBV); hepatitis C virus (HCV), hepatitis E virus (HEV), hepatitis G and GB virus (GBV-C); human immunodeficiency viruses (HIV); human papilloma viruses (HPV); herpes simplex viruses (HSV); Molluscum contagiosum virus (MCV); influenza virus; Epstein-Barr virus (EBV) and varicella-zoster virus (VZV).

8. The method any of any previous claim, wherein the microorganisms are all pathogens and the DMP is directed towards identification of microorganisms that are associated with sexually transmitted infection.

9. The method of claim 8, wherein the DMP comprises primer sequences that hybridise with one or more target sequences obtained from microorganisms selected from the group consisting of: Mycoplasma genitalum; Mycoplasma hominis; Chlamydia trachomatis.; Ureaplasma urealyticum; Neisseria gonorrhoea; Gardnerella vaginalis; Trichomonas vaginalis.; Treponema pallidum; and Candida albicans.

10. The method of any previous claim wherein the highly conserved polymorphic allele comprises all or a part of a microorganism gene selected from: a bacterial 16S rRNA; a bacterial 32S rRNA; a yeast 16S rRNA; a yeast 18S rRNA; and a viral polymerase.

11. The method of any previous claim, wherein if two or more specified microorganisms are present in the biological sample the primer extension reaction produces a DMP reaction product comprising at least two extended primer sequences each of a known predetermined molecular weight that is different to the other.

12. The method of claim 11, wherein DMP reaction product(s) are analysed using a technique that resolves extended primer sequences according to their molecular weight.

13. The method of any of claims 1 to 10, wherein the primer extension reaction comprises a primer labelling reagent such that if a specified microorganisms is present in the biological sample the primer extension reaction incorporates the labelling reagent into the primer extension product, thereby producing a DMP reaction product comprising the labelling reagent, and wherein DMP reaction product(s) are analysed using a technique that identifies presence of an incorporated labelling reagent in the primer extension product.

14. The method of any previous claim, wherein the plurality of primer sequences comprised within the DMP are immobilized on a solid support.

15. The method of any previous claim, wherein the plurality of amplification primers comprise one or more primer pairs selected from the group consisting of SEQ ID NOs: 1/2, 3/4, 5/6, 7/8, 9/10, 11/12, 13/14, 15/16, 17/18, 19/20, 21/22, 23/24, 25/26, 27/28 and 29/30; and/or one or more primer pairs selected from the group consisting of SEQ ID NOs: 46/47; 48/49; 50/51; 52/53; 54/55; 56/57; 58/59; 60/61; 62/63; 64/65; 66/67; 68/69; 70/71; 72/73; and 74/75.

## Patentansprüche

1. Verfahren zur Bestimmung, ob zwei oder mehr Spezies spezifischer Mikroorganismen innerhalb einer biologischen, potenziell die Mikroorganismen enthaltenden Probe vorhanden sind, umfassend:
(a) Immobilisieren der biologischen Probe an einem ersten Ort auf einem und/oder innerhalb eines festen Substrats;
(b) Überführen der immobilisierten, biologischen Probe an mindestens einen zweiten Ort und Durchführen eines Extraktionsschritts an dem festen Substrat, um jegliche Mikroorganismus-DNA zu extrahieren, die auf dem und/oder innerhalb des festen Substrats immobilisiert ist;
(c) Durchführen eines Nukleinsäureamplifizierungsschritts an der in Schritt (b) extrahierten Mikroorganismus-DNA, wobei der Amplifizierungsschritt eine Vielzahl von Amplifizierungsprimern umfasst, die auf eine Amplifizierung einer Vielzahl von hoch konservierten Sequenzen aus den zwei oder mehr spezifischen Mikroorganismen gerichtet sind, und wobei die amplifizierten Sequenzen als Zielsequenzen bezeichnet werden;
(d) Kombinieren der Zielsequenzen mit einer Vielzahl von Primersequenzen, die in einer Mehrfach-Diagnose-Platte (MDP) enthalten sind, wobei jede Primersequenz die Genotypisierung der Zielsequenz erleichtert;
(e) Durchführen einer Primerverlängerungsreaktion an der in (d) vorhandenen Kombination aus Zielsequenzen und der MDP, wodurch ein MDP-Reaktionsprodukt erzeugt wird; und
(f) Analysieren des Reaktionsprodukts, um den Genotyp von beliebigen, vorhandenen Zielsequenzen zu bestimmen, und Korrelieren des Genotyps der Zielsequenzen im Reaktionsprodukt mit der Identifizierung von spezifischen Mikroorganismen, die in der biologischen Probe vorhanden sind;
wobei die spezifischen Mikroorganismen ausgewählt werden aus einem oder mehreren der Gruppe bestehend aus: Mykoplasma spp., Chlamydia spp., Ureaplasma spp., Neisseria spp., Gardnerella spp., Trichomonas spp. und Treponema spp. und Candida albicans;
und wobei die MDP eine oder mehrere Primersequenzen umfasst, die ausgewählt werden aus SEQ ID NO.: 31-45, und/oder einer oder mehreren Primersequenzen, die ausgewählt werden aus SEQ ID NO.: 76-90.

2. Verfahren nach Anspruch 1, wobei der erste Ort von dem zweiten Ort entfernt liegt.

3. Verfahren nach Anspruch 1 oder Anspruch 2, wobei die biologische Probe mindestens eine umfasst aus der Gruppe bestehend aus: Urin, Speichel, Blut, Sputum; Semen; Faeces; Nasalabstrich, Tränen, Vaginalabstrich, Rektalabstrich, Zervikalabstrich, Gewebebiopsie und Harnröhrenabstrich.

4. Verfahren nach einem der vorhergehenden Ansprüche, wobei das feste Substrat ein absorbierendes Fasermaterial umfasst, das mit einem oder mehreren Reagenzien imprägniert ist, die jeglichen, in der biologischen Probe vorhandenen Mikroorganismus immobilisieren und inaktivieren.

5. Verfahren nach Anspruch 4, wobei das feste Substrat ein Material umfasst, ausgewählt aus einem cellulosebasierten Papier, einer Mikrofaser-Membran, einem Glasfasermaterial, einem Polymerfasermaterial, einem Gewebe und einem Vlies; und/oder wobei das feste Substrat Whatman FTA^{®} oder Whatman FTA^{®} Elute-Reagenz oder Whatman FTA^{®} Elute-Papier umfasst.

6. Verfahren nach einem der vorhergehenden Ansprüche, wobei die MDP auf die Identifizierung von Allelen aus einer Kombination von Mikroorganismen gerichtet ist, die potenziell in der biologischen Probe vorhanden sind, wobei die Kombination Bakterien, Pilze und Viren umfasst.

7. Verfahren gemäß Anspruch 6, wobei die Viren ausgewählt werden aus der Gruppe bestehend aus: Zytomegalievirus (CMV); Hepatitis-A-Virus (HAV); Hepatitis-B-Virus (HBV); Hepatitis-C-Virus (HCV), Hepatitis-E-Virus (HEV), Hepatitis-G- und GB-Virus (GBV-C); Humane Immundefizienz-Viren (HIV); Humane Papilloma-Viren (HPV); Herpes-simplex-Viren (HSV); Molluscum-contagiosum-Virus (MCV); Influenza-Virus, Epstein-Barr-Virus (EBV) und Varizella-zoster-Virus (VZV).

8. Verfahren nach einem der vorhergehenden Ansprüche, wobei die Mikroorganismen alles Pathogene sind und die MDP auf die Identifizierung von Mikroorganismen gerichtet ist, die mit einer sexuell übertragbaren Infektion im Zusammenhang stehen.

9. Verfahren nach Anspruch 8, wobei die MDP Primersequenzen umfasst, die mit einer oder mehreren Zielsequenzen hybridisieren, die von Mikroorganismen erhalten wurden, die ausgewählt werden aus der Gruppe bestehend aus: Mycoplasma genitalum, Mycoplasma hominis, Chlamydia trachomatis, Ureaplasma urealyticum, Neisseria gonorrhoea, Gardnerella vaginalis, Trichomonas vaginalis, Treponema pallidum und Candida albicans.

10. Verfahren nach einem der vorhergehenden Ansprüche, wobei die hoch konservierten, polymorphen Allele das gesamte oder einen Teil eines Mikroorganismusgens umfassen, ausgewählt aus: einer bakteriellen 16S-rRNA; einer bakteriellen 32S-rRNA; einer Hefe-16S-rRNA; einer Hefe-18S-rRNA und einer viralen Polymerase.

11. Verfahren nach einem der vorhergehenden Ansprüche, wobei die Primerverlängerungsreaktion, wenn zwei oder mehr spezifische Mikroorganismen in der biologischen Probe vorhanden sind, ein MDP-Reaktionsprodukt erzeugt, das mindestens zwei verlängerte Primersequenzen mit jeweils einem vorbestimmten Molekulargewicht umfasst, das sich von dem anderen unterscheidet.

12. Verfahren nach Anspruch 11, wobei das/die MDP-Reaktionsprodukt(e) unter Verwendung einer Technik analysiert wird/werden, welche die verlängerten Primersequenzen nach ihrem Molekulargewicht auflöst.

13. Verfahren nach einem der Ansprüche 1 bis 10, wobei die Primerverlängerungsreaktion ein Primermarkierungsreagenz umfasst, sodass bei Vorhandensein eines spezifischen Mikroorganismus in der biologischen Probe die Primerverlängerungsreaktion das Markierungsreagenz in das Primerverlängerungsprodukt einbaut, wodurch ein das Markierungsreagenz enthaltendes MDP-Reaktionsprodukt erzeugt wird, und wobei das/die MDP-Reaktionsprodukt(e) unter Verwendung einer Technik analysiert wird/werden, welche das Vorhandensein des eingebauten Markierungsreagenzes in dem Primerverlängerungsprodukt identifiziert.

14. Verfahren nach einem der vorhergehenden Ansprüche, wobei die innerhalb der MDP enthaltene Vielzahl von Primersequenzen auf einem festen Träger immobilisiert ist.

15. Verfahren nach einem der vorhergehenden Ansprüche, wobei die Vielzahl von Amplifizierungsprimern ein oder mehrere Primerpaare umfasst, die ausgewählt werden aus der Gruppe bestehend aus SEQ ID NO.: 1/2, 3/4, 5/6, 7/8, 9/10, 11/12, 13/14, 15/16, 17/18, 19/20, 21/22, 23/24, 25/26, 27/28 und 29/30; und/oder ein oder mehrere Primerpaare, die ausgewählt werden aus der Gruppe bestehend aus SEQ ID NO.: 46/47; 48/49; 50/51; 52/53; 54/55; 56/57; 58/59; 60/61; 62/63; 64/65; 66/67; 68/69; 70/71; 72/73 und 74/75.

## Revendications

1. Méthode permettant de déterminer si deux ou plusieurs espèces de microorganismes spécifiés sont présentes dans un échantillon biologique susceptible de contenir ces microorganismes, qui comprend les étapes consistant à :
(a) immobiliser l'échantillon biologique sur et/ou dans un substrat solide en une première localisation ;
(b) transférer l'échantillon biologique immobilisé en au moins une seconde localisation et réaliser une étape d'extraction sur le substrat solide de manière à extraire tout ADN de microorganisme immobilisé sur et/ou dans le substrat solide ;
(c) réaliser une étape d'amplification de l'acide nucléique sur l'ADN de microorganisme extrait à l'étape (b), l'étape d'amplification comprenant une pluralité d'amorces d'amplification qui visent à produire l'amplification d'une pluralité de séquences hautement conservées à partir des deux ou plusieurs microorganismes spécifiques, et les séquences amplifiées étant désignées sous le nom de séquences cibles ;
(d) combiner les séquences cibles à une pluralité de séquences amorces comprises dans un système de multiplexage diagnostique (DMP, pour *diagnostic multiplexing panel*), où chaque séquence amorce facilite le génotypage de la séquence cible ;
(e) réaliser une réaction d'allongement de l'amorce sur la combinaison de séquences cibles et le DMP présente dans (d), donnant ainsi un produit de réaction du DMP ; et
(f) analyser le produit de réaction pour déterminer le génotype de toute séquence cible présente et corréler le génotype des séquences cibles obtenues dans le produit de réaction avec l'identification de microorganismes spécifiés présents dans l'échantillon biologique ;
dans laquelle les microorganismes spécifiés sont sélectionnés parmi un ou plusieurs de ceux du groupe consistant en : Mycoplasma spp., Chlamydia spp., Ureaplasma spp., Neisseria spp., Gardnerella spp., Trichomonas spp., Treponema spp. et Candida albicans ;
et dans laquelle le DMP comprend une ou plusieurs séquences amorces sélectionnées parmi les SÉQ ID N° : 31-45 et/ou une ou plusieurs séquences amorces sélectionnées parmi les SÉQ ID N° : 76-90.

2. Méthode de la revendication 1, dans laquelle la première localisation est à distance de la seconde localisation.

3. Méthode de la revendication 1 ou de la revendication 2, dans laquelle l'échantillon biologique comprend au moins un de ceux du groupe consistant en les suivants : urine ; salive ; sang ; expectorations ; sperme ; matières fécales ; prélèvement par écouvillonnage nasal ; larmes ; prélèvement par écouvillonnage vaginal ; prélèvement par écouvillonnage rectal ; frottis cervical ; biopsie tissulaire ; et prélèvement par écouvillonnage urétral.

4. Méthode de l'une quelconque des revendications précédentes, dans laquelle le substrat solide comprend un matériau fibreux absorbant imprégné d'un ou de plusieurs réactifs qui produisent l'immobilisation et l'inactivation de tout microorganisme présent dans l'échantillon biologique.

5. Méthode de la revendication 4, dans laquelle le substrat solide comprend un matériau sélectionné parmi les suivants : papier à base de cellulose ; membrane microfibreuse ; matériau à base de fibres de verre ; matériau à base de fibres polymères ; textile tissé ; et textile non tissé ; et/ou dans laquelle le substrat solide comprend un réactif Whatman FTA^{®} ou Whatman FTA^{®} Elute ou un papier Whatman FTA^{®} Elute.

6. Méthode de l'une quelconque des revendications précédentes, dans laquelle le DMP est axé sur l'identification d'allèles à partir d'une combinaison de microorganismes potentiellement présents dans l'échantillon biologique, la combinaison incluant des bactéries, espèces fongiques et virus.

7. Méthode de la revendication 6, dans laquelle les virus sont sélectionnés dans le groupe consistant en les suivants : cytomégalovirus (CMV) ; virus de l'hépatite A (VHA); virus de l'hépatite B (VHB); virus de l'hépatite C (VHC), virus de l'hépatite E (VHE), virus de l'hépatite G et GB (VHG et VHGB) ; virus de l'immunodéficience humaine (VIH) ; papillomavirus humains (PVH); virus Herpes simplex (VHS) ; virus du molluscum contagiosum (MCV) ; virus de la grippe ; virus Epstein-Barr (VEB) et herpesvirus varicellae (HVV).

8. Méthode de l'une quelconque des revendications précédentes, dans laquelle les microorganismes sont tous pathogènes et le DMP est axé sur l'identification de microorganismes qui sont associés à une infection transmise sexuellement.

9. Méthode de la revendication 8, dans laquelle le DMP comprend des séquences amorces qui s'hybrident à une ou plusieurs des séquences cibles obtenues à partir de microorganismes sélectionnés dans le groupe consistant en les suivants : Mycoplasma genitalum ; Mycoplasma hominis ; Chlamydia trachomatis ; Ureaplasma urealyticum ; Neisseria gonorrhoea ; Gardnerella vaginalis ; Trichomonas vaginalis ; Treponema pallidum ; et Candida albicans.

10. Méthode de l'une quelconque des revendications précédentes, dans laquelle l'allèle polymorphe hautement conservé comprend la totalité ou une partie du gène d'un microorganisme sélectionnée parmi les suivantes : ARNr 16S bactérien ; ARNr 32S bactérien ; ARNr 16S d'une levure ; ARNr 18S d'une levure ; et polymérase virale.

11. Méthode de l'une quelconque des revendications précédentes, dans laquelle la présence de deux ou plusieurs microorganismes spécifiés dans l'échantillon biologique fait que la réaction d'allongement de l'amorce donne un produit de réaction du DMP comprenant au moins deux séquences amorces allongées, chacune d'un poids moléculaire prédéterminé connu qui diffère de celui de l'autre.

12. Méthode de la revendication 11, dans laquelle le(s) produit(s) de réaction du DMP est/sont analysé(s) en utilisant une technique qui permet de résoudre les séquences amorces allongées en fonction de leur poids moléculaire.

13. Méthode de l'une quelconque des revendications 1 à 10, dans laquelle la réaction d'allongement de l'amorce comprend un réactif de marquage de l'amorce tel que si un microorganisme spécifié est présent dans l'échantillon biologique, la réaction d'allongement de l'amorce incorpore le réactif de marquage dans le produit d'allongement de l'amorce, donnant ainsi un produit de réaction du DMP qui comprend le réactif de marquage, et dans laquelle le(s) produit(s) de réaction du DMP est/sont analysé(s) en utilisant une technique qui permet d'identifier la présence d'un réactif de marquage incorporé dans le produit d'allongement de l'amorce.

14. Méthode de l'une quelconque des revendications précédentes, dans laquelle la pluralité de séquences amorces comprises dans le DMP est immobilisée sur un support solide.

15. Méthode de l'une quelconque des revendications précédentes, dans laquelle la pluralité d'amorces d'amplification comprend une ou plusieurs paires d'amorces sélectionnées dans le groupe consistant en les SÉQ ID N° : 1/2, 3/4, 5/6, 7/8, 9/10, 11/12, 13/14, 15/16, 17/18, 19/20, 21/22, 23/24, 25/26, 27/28 et 29/30 ; et/ou une ou plusieurs paires d'amorces sélectionnées dans le groupe consistant en les SÉQ ID N° : 46/47, 48/49, 50/51, 52/53, 54/55, 56/57, 58/59, 60/61, 62/63, 64/65, 66/67, 68/69, 70/71, 72/73 et 74/75.
